# EUROPÄISCHE PATENTSCHRIFT

(11) **EP 1 685 789 B1**
(45) Veröffentlichungstag und Bekanntmachung des Hinweises auf die Patenterteilung: **20.10.2010**
(21) Anmeldenummer: 05026838.2
(22) Anmeldetag: 08.12.2005
(51) Int. Cl.: A61B 1/01

(54) **Endoskop mit längsgeführtem Stülpschlauch**
Endoscope with axially guided everted tube
Endoscope avec un tube retourné, guidé longitudinalement

(30) Priorität: 01.02.2005 DE 102005004622
(43) Veröffentlichungstag der Anmeldung: 02.08.2006
(73) Patentinhaber: Invendo Medical GmbH, 69469 Weinheim (DE)
(72) Erfinder: Bob, Dr. Konstantin, 69469 Weinheim (DE)
(74) Vertreter: Winter, Brandl, Fürniss, Hübner, Röss, Kaiser, Polte - Partnerschaft

(56) Entgegenhaltungen:
- EP-A- 1 477 105
- WO-A-00/48506
- WO-A-99/51153
- US-A- 4 561 427
- US-A- 5 259 364
- US-A- 5 571 114
- US-A- 5 792 114
- US-A1- 2001 044 595
- US-A1- 2004 204 702

## Beschreibung

Die vorliegende Erfindung betrifft eine Konstruktion eines Endoskops mit einen Stülpschlauch und einem Endoskopschaft, die jeweils mit einer Längsführungseinrichtung vorgesehen sind. Insbesondere betrifft die vorliegende Erfindung ein Endoskop mit einem geführten Stülpschlauch, der mit dem Endoskopschaft dadurch in Gleiteingriff gebracht wird, dass eine der Längsführungseinrichtungen eine Hinterschneidung ausbildet, welche mit der entsprechend geformten anderen Längsführungseinrichtung in Gleiteingriff steht. Weiter betrifft die vorliegende Erfindung ein Endoskop, bei welchem die Längsführungseinrichtungen jeweils als ein Stetigförderer am Endoskopschaft und als eine Eingriffseinrichtung am Stülpschlauch ausgebildet sind.

Endoskope werden im Wesentlichen zur visuellen Untersuchung der Speiseröhre, des Magens, des Darms vom Magen oder vom Anus aus, der Harnröhre sowie der Blase eingesetzt. Hierfür ist das Endoskop an seinem distalen Ende mit einer Beleuchtungseinrichtung sowie mit einer Optik, vorzugsweise einem Kamerachip, ausgestattet, der über Leitungen innerhalb eines Endoskopschafts mit einer Kamerasteuerung am proximalen Ende des Endoskopschafts verbunden ist. Die Kamerasteuerung ist wiederum über einen Videoprozessor mit einem externen Monitor verbunden, auf dem der behandelnde Arzt die zu untersuchenden Bereiche erkennen kann. Das in den Hohlkanal einzuführende distale Ende des Endoskopschafts ist dabei in jede Richtung abkrümmbar ausgebildet und lässt sich mittels einer Handhabe, vorzugsweise über zwei Steuerräder mit Bremse am hinteren Endabschnitt des Endoskops manuell ähnlich eines Fingers abwinkeln. Des Weiteren ist in der Regel der Endoskopschaft mit zumindest zwei Kanälen durchzogen, die sich an der vordersten Spitze des distalen Endes öffnen. Durch diese Kanäle kann bei Bedarf einmal Reinigungsfluid zum Säubern einer zu untersuchenden Stelle oder Co2 (Luft) zum Entfalten des Hohlkanals gepresst oder zum Ändern über einen Arbeitskanal diverse Arbeitsgerätschaften, beispielsweise Zangen oder Scheren zur Entnahme von Gewebeproben, Biopsienadeln, aufheizbare Schneiddrähte, Koagulationselektroden, geschoben werden, die am proximalen Ende des Endoskopschafts ebenfalls über Bedienungsdrähte oder Bowdenzüge innerhalb des inneren Kanals manuell betätigt werden können. Bei einer Gewebeprobeentnahme wird nachdem das distale Ende die betreffende Stelle erreicht hat, beispielsweise eine Zange am proximalen Ende des Endoskopschafts in den Kanal eingeführt und zum distalen Ende vorgeschoben. Nach erfolgter Entnahme der Probe wird die Zange wieder zurückgezogen und aus dem Kanal entfernt, so dass mit der weiteren Untersuchung fortgefahren werden kann.

Im allgemeinen hat das Endoskop eine langgestreckte schlauchförmige Gestalt mit einem Durchmesser von ca. 9 bis 15 mm und besteht aus einem biegsamen Material, um Krümmungen des zu untersuchenden Hohlkanals, beispielsweise Darmwindungen, folgen zu können. Jedoch beult das herkömmliche Endoskop, trotz langgestreckter, schlauchförmiger Gestalt bei starken Krümmungen des Darms diesen im gewissen Rahmen aus, um sich dem Verlauf des Darms bei der Einführbewegung bzw. bei der fortschreitenden Bewegung des Endoskops in den Hohlkanal anzupassen. Dies kann für den zu untersuchenden Patienten sehr schmerzhaft sein.

Daher wurde ein aus dem Stand der Technik, wie z.B der DE 197 48 500 A1, bekanntes Endoskop entwickelt, welches einen Stülpschlauch für einen inneren Schaft verwendet. Dieser Stülpschlauch kann an dessen beiden Enden oder auch nur an einem Ende gestülpt sein, wodurch sich der Stülpschlauch in einen radial äußeren Abschnitt, einen Umstülpabschnitt und einen radial inneren Abschnitt einteilen lässt. Der radial äußere Abschnitt des Stülpschlauchs steht mit einer Wandung des Hohlkanals, beispielsweise der Darmwand, in Berührung bzw. liegt an dieser an. Somit befindet sich der radial äußere Abschnitt des Stülpschlauchs in einer Ruhelage, bzw. in einem nicht bewegten Zustand relativ zum Endoskopschaft, wie nachstehend beschrieben wird. Der radial innere Abschnitt des Stülpschlauchs befindet sich mit einem in den Stülpschlauch eingeführten Schaft (Endoskopschaft) in Berührung, wobei bei Bewegung des Endoskopschafts der radial innere Abschnitt in Bewegung versetzt wird, während der radial äußere Abschnitt sich in der Ruhelage befindet.

Um den Endoskopschaft bzw. den Stülpschlauch in dem Hohlkanal fortzubewegen ist unter Anderem ein sogenannter Stülpschlauchantrieb bekannt, wie er bspw. in der DE 199 20717 A1 und der DE 197 17 108 A1 offenbart ist.

Bei dieser Art von Antrieb befindet sich an dem Stülpschlauch eine Antriebseinrichtung, die den radial inneren Abschnitt des Stülpschlauchs in eine Einführrichtung bei gleichzeitiger Ruhelage des radial äußeren Abschnitts des Stülpschlauchs voranbewegt. Da der Endoskopschaft mit dem radial inneren Abschnitt des Stülpschlauchs in Berührung steht, wird dieser zusammen mit dem Stülpschlauch voranbewegt. Somit wird der Endoskopschaft durch den Stülpschlauch in Bewegung versetzt, wobei sich der Stülpschlauch durch eine quasi "Abrollbewegung" den Krümmungen des Hohlkanals, beispielsweise des Darms, anpasst und sich an diesen anschmiegt. Dadurch wird ein Ausbeulen des Darms aufgrund der quasi "Abrollbewegung" des Stülpschlauchs verhindert. Da der radial äußere Abschnitt des Stülpschlauchs an der Wandung des Hohlkanals, beispielsweise der Darmwand, anliegt und sich somit in Ruhelage befindet und der radial innere Abschnitt des Stülpschlauchs durch seine quasi "Abrollbewegung" voranbewegt wird, bewegt sich der Endoskopschaft zwangsweise nach den Gesetzen der Kinematik schneller, als der Umstülpabschnitt des Stülpschlauchs voranschreiten kann. Um diese Geschwindigkeitsdifferenz zu kompensieren, sind in der Lösung des Stands der Technik ein vorderes und ein hinteres Anschlagstück an dem Endoskopschaft angebracht, zwischen welchen der Stülpschlauch angeordnet ist. Dadurch kann verhindert werden, dass der Endoskopschaft dem Stülpschlauch vorauseilt und wie bei einem gewöhnlichem Endoskop die Darmwand ausbeult.

Jedoch liegt der Lösung des Stands der Technik das Problem zugrunde, dass durch die Verwendung solcher Anschlagstücke eine erhöhte Reibung zwischen diesen und dem Stülpschlauch auftritt, wodurch sich die Antriebsleistung der Antriebseinrichtung verschlechtert. Weiter ist es durch die oben beschriebene Anordnung nicht möglich, den Endoskopschaft eigenständig bzw. relativ zum dem Stülpschlauch zu bewegen.

Weiter ist aus dem Stand der Technik, beispielsweise gemäß der DE-US 199 20 717, eine Stülpschlauchkonstruktion der vorstehend genannten Gattung bekannt. Diese bekannte Konstruktion hat einen am distalen sowie proximalen Endabschnitt eines Endoskops umgestülpten Schlauch vorzugsweise, bestehend aus einem Silikonmaterial, der einen Endoskopschaft zumindest in seinem Mittenabschnitt ummantelt. Vorzugsweise ist eine Antriebseinrichtung vorgesehen, welche den Stülpschlauch in Längsrichtung des Endoskops antreibt. Hierfür hat die Antriebseinrichtung ein Gehäuse, an dem die sich gegenüberliegenden freien Enden des radial äußeren Abschnitts des Stülpschlauchs fixiert sind und aus dem Kraftübertragungsmittel beispielsweise in Form von Antriebsrädern, Raupen oder Schuhen herausragen, welche eine Antriebskraft auf den radial inneren Abschnitt des Stülpschlauchs und somit teilweise auch auf den sich darin befindlichen Endoskopschaft ausüben.

Der Stülpschlauch überträgt diese Antriebskraft teilweise auf den Endoskopschaft, indem sich der distale/proximale Endabschnitt des Stülpschlauchs an einem am Endoskopschaft vorgesehenen radialen Vorsprung gleitfähig axial abstützt und somit bei einer Ausstülpbewegung den Endoskopschaft vorwärts drückt. Um ein Aufschuppen oder Verwerfungen des Stülpschlauchs, hervorgerufen durch die resultierenden Kräfte, zu verhindern, weist der bekannte Stülpschlauch eine zusätzliche Armierung beispielsweise in Form einer einlaminierten Spiralfeder oder eines Gewebes auf. Auch ist es bekannt, den vom Stülpschlauch und dem Antriebsgehäuse gebildeten Hohlraum mit einem Fluid, beispielsweise einem Öl, druckzubefüllen und dadurch einen Reibungsverlust zu reduzieren und gleichzeitig den Stülpschlauch aufzublähen.

All diese Maßnahmen haben jedoch das Problem, dass sie die Flexibilität des gesamten Endoskops verringern und dadurch dessen minimal erreichbaren Biegeradius vergrößern.

Schließlich ist aus dem Stand der Technik bereits bekannt, einen Stülpschlauchabschnitt an seinem distalen Umstülpabschnitt zwangszuführen. Der mit diesem Stülpschlauch ausgerüstete Endoskopschaft weist hierfür an seinem distalen Endabschnitt eine radial umlaufende Hinterschneidung auf, die vom distalen Umstülpabschnitt des Stülpschlauchs überlagert ist. Innerhalb des Stülpschlauchs ist im Bereich des distalen Umstülpabschnitts ein Schnappring eingelegt, der sich im montierten Zustand des Endoskops hinter der Hinterschneidung des Endoskopschafts anordnet und somit ein Zurückziehen des distalen Umstülpabschnitts verhindert. Der Schnappring ist dabei mit Spiel innerhalb des distalen Umstülpabschnitts gelagert, um ein Durchrutschen des Stülpschlauchs bei einer Vortriebsbewegung des Schafts zu ermöglichen. Zusätzlich ist im distalen Endabschnitt des Endoskopschafts optional ein Permanentmagnet angeordnet, der eine externe magnetische Anziehungskraft auf den Schnappring ausübt und somit den Stülpschlauch in Längsrichtung streckt.

Durch die vorstehend beschriebene Ausgestaltung lässt sich zwar ein Aufschuppen des Stülpschlauchs verhindern, indessen haftet einer solchen Lösung das Problem einer hohen Reibung zwischen Schnappring und Stülpschlauch an, wodurch die auf den Stülpschlauch aufzubringende Antriebskraft ansteigt.

Die US 2004/0204702 A1 offenbart ein Endoskop mit einem Endoskopschaft, in dem Antriebsrollen gelagert sind, die über dessen Außenseite hervorstehen, um mit einer radial inneren Seite eines eine Blase bildenden und den Endoskopschaft umgebenden Stülpschlauchs einzugreifen, um den Stülpschlauch anzutreiben.

Daher ist es eine Aufgabe der vorliegenden Erfindung, eine Konstruktion eines Endoskops bereitzustellen, das die vorstehend erwähnten Problematiken lösen kann und ebenso eine nahezu schmerzfreie Untersuchung des Patienten gewährleistet.

Ein Ziel der vorliegenden Erfindung ist es dabei, ein Endoskop mit einer Stülpschlauchkonstruktion bereitzustellen, welche geringe Aufschubtendenzen zeigt, wobei die Flexibilität des Endoskops nicht über Gebühr verringert wird.

Die vorstehend genannte Aufgabe wird durch ein Endoskop mit den Merkmalen des Anspruchs 1 gelöst. Weitere vorteilhafte Ausgestaltungen der vorliegenden Erfindung werden durch die Merkmale der übrigen Unteransprüche erzielt.

Der Grundgedanke der Erfindung besteht demzufolge im Wesentlichen darin, dass das Endoskop mit einem Endoskopschaft einen aus einem radial äußeren Abschnitt und einem radial inneren Abschnitt bestehenden Stülpschlauch aufweist, wobei der Endoskopschaft von dem radial inneren Abschnitt des Stülpschlauchs zumindest teilweise umgeben wird und Längsführungseinrichtungen jeweils an dem Endoskopschaft und/oder dem Stülpschlauch ausgebildet sind, die miteinander in Eingriff stehen. Ein an dem vorstehend genannten Grundgedanken anlehnendes Erfindungskonzept besteht darin, dass die jeweiligen Längsführungseinrichtungen zum Einen als ein an dem Endoskopschaft in dessen Längsrichtung verlaufender Stetigförderer und zum Anderen als eine mit dem Stetigförderer in Form- oder Reibschluß stehende Eingriffseinrichtung ausgebildet sind, die an dem Stülpschlauch, noch genauer an einer Stülpschlauchseite, vorgesehen ist. Hierbei wird ebenso ein Eingriff des Stülpschlauchs mit dem Endoskopschaft in Bewegungslängsrichtung des Endoskopschafts hergestellt, wobei in diesem Fall der Stetigförderer eine sich selbst bewegende Längsführungseinrichtung darstellt (aktiv).

Die Erfindung wird nachstehend anhand bevorzugter Ausführungsbeispiele unter Bezugnahme auf die begleitenden Zeichnungen näher erläutert.
Fig. 1 zeigt ein Endoskop mit Stülpschlauch und Endoskopschaft gemäß einem ersten Ausführungsbeispiel der vorliegenden Erfindung,
Fig. 2 zeigt ein Endoskop gemäß dem ersten Ausführungsbeispiel der vorliegenden Erfindung, wobei zusätzlich ein Stülpschlauchantrieb für das Endoskop vorgesehen ist,
Fig. 3 zeigt ein Endoskop gemäß einem zweiten Ausführungsbeispiel der vorliegenden Erfindung, wobei zusätzlich ein Schaftantrieb für das Endoskop vorgesehen ist,
Fig. 3a zeigt eine Ausführungsform des Stetigförderers,
Fig. 4 zeigt ein drittes Ausführungsbeispiel eines erfindungsgemäßen Endoskops,
Fig. 5 zeigt die Perspektivenansicht eines Stülpschlauchs gemäß einem Beispiel, das durch die Erfindung nicht abgedecht ist,
Fig. 6 zeigt die Querschnittsansicht des Stülpschlauchs von Fig. 5,
Fig. 7 zeigt einen perspektivischen Ausschnitt eines radialen Vorsprungs eines Endoskopschafts gemäß einem ersten Beispiel, das durch die Erfindung nicht abgedeckt ist,
Fig. 8 zeigt die Perspektivenansicht eines Mittenabschnitts des Endoskopschafts mit montiertem Stülpschlauch gemäß einem ersten Beispiel, das durch die Erfindung nicht abgedeckt ist,
Fig. 9 zeigt die Perspektivenansicht eine Stülpschlauchs gemäß einem einem zweiten Beispiel, das durch die Erfindung nicht abgedeckt ist,
Fig. 10 zeigt die Querschnittsansicht des Stülpschlauchs gemäß einem zweiten Beispiel, das durch die Erfindung nicht abgedeckt ist, und
Fig. 11 zeigt einen radialen Vorsprung des Endoskopschafts gemäß einem zweiten Beispiel, das durch die Erfindung nicht abgedeckt ist.

Erfindungsgemäß wird ein Endoskop 1 bereitgestellt, das allgemein einen Endoskopschaft 3 und einen Stülpschlauch 2 aufweist und das für die Untersuchung eines kanalartigen Hohlraums verwendet wird. Der Endoskopschaft 3, der ein in Einführrichtung vorangehendes Ende, das distale Ende, und ein nachlaufendes Ende, das proximale Ende, hat, weist weiter zumindest eine optische Einrichtung zum optischen Erfassen des zu untersuchen Hohlraums auf und kann weiter wahlweise eine Beleuchtungseinrichtung, eine Behandlungseinrichtung, eine Sprüheinrichtung usw. aufweisen (sämtlich in den Figuren nicht dargestellt). Der Stülpschlauch 3 besteht wie aus dem Stand der Technik bereits bekannt ist, aus einem radial inneren Abschnitt 4 der sich an zumindest einem distalen Umstülpabschnitt zu einem radial äußeren Abschnitt 5 verlängert. Um den Ansprüchen einer hohen Biegsamkeit zu genügen, werden sowohl für den Endoskopschaft als auch für den Stülpschlauch vorzugsweise hochflexible Materialen verwendet, wie beispielsweise EPTFE, wodurch sich der Stülpschlauch 2 und der Endoskopschaft 3 leicht dem Verlauf des zu untersuchenden Kanals, beispielsweise dem Darm eines Patienten, anpassen können. Der Endoskopschaft 3 ist von dem radial inneren Abschnitt 4 des Stülpschlauchs zumindest teilweise unmittelbar umgebenen, wobei sich in Längsrichtung des Endoskopschafts erstreckende Längsführungseinrichtungen jeweils an dem Endoskopschaft 3 und dem Stülpschlauch 2 ausgebildet sind, die miteinander in Eingriff stehen.

Der radial äußere Abschnitt 5 des Stülpschlauchs 2 liegt bei der Einführung in den Hohlkanal, beispielsweise dem Darm, an der Wandung des Hohlkanals, beispielsweise der Darmwand, an und befindet sich somit fortwährend in einer Ruhestellung bzw. in einer Ruhelage bezüglich des Endoskopschafts 3. Der radial innere Abschnitt 4 des Stülpschlauchs 2 steht mit dem Endoskopschaft 3 in Kontakt. Weiter ist der radial innere Abschnitt 4 derjenige, der sich relativ zu dem radial äußeren Abschnitt 5 mit einer bestimmten Geschwindigkeit bewegt. Durch das Umstülpen des Schlauchs 2 wird der radial innere Abschnitt 4 bei Bewegung in Richtung des Verlaufs des Hohlkanals und gleichzeitiger Ruhestellung des radial äußeren Abschnitts 5 durch eine quasi "Abrollbewegung" voranbewegt und somit in den radial äußeren Abschnitt überführt.

Gemäß dem ersten Ausführungsbeispiel der Erfindung besteht zwischen dem radial inneren Abschnitt 4 des Stülpschlauchs 2 und dem Endoskopschaft 3 eine Wirkverbindung, die sich zum Einen aus einer Eingriffseinrichtung 6 und zum Anderen aus einem an dem Endoskopschaft 3 befindlichen Stetigförderer 7 zusammensetzt, welcher in Längsrichtung des Endoskopschafts verläuft und als eine Längsführungseinrichtung fungiert. Die Eingriffseinrichtung 6 besteht dabei aus zwei ineinander greifenden Komponenten die am Stülpschlauch bzw. am Stetigförderer 7 angeordnet sind. Die an dem Stülpschlauch befindliche Komponente der Eingriffseinrichtung 6 ist lediglich an einer Seite des Stülpschlauchs angebracht, nämlich an der Außenseite des Stülpschlauchs und erstreckt sich zumindest abschnittsweise längs des radial inneren Schlauchabschnitts und ggf. auch des radial äußeren Schlauchabschnitts.

Die Eingriffseinrichtung 6 kann dabei in unterschiedlichen Ausführungsformen ausgebildet sein, so zum Beispiel als eine formschlüssige, eine reibschlüssige Ausführungsform oder kann auch eine Mischform von den beiden vorstehend genannte Ausführungsformen sein, wodurch der Eingriff mit dem entsprechend ausgeführten Stetigförderer hergestellt wird. Beispielsweise kann die Eingriffseinrichtung eine Mischform wie ein Klettverschluss sein, wobei an der Außenseite des Stülpschlauchs ein längsverlaufendes Klettband angeordnet ist, während der Stetigförderer in diesem Fall mit der entsprechenden Gegenkomponente zum Klettband ausgerüstet ist. Es ist aber auch denkbar, die Eingriffseinrichtung aus Vor- und Rücksprüngen auszubilden, welche wahlweise oder abwechselnd im Stülpschlauch und am Stetigförderer angeordnet sind und formschlüssig sowie flexibel und damit lösbar ineinander greifen.

Die einfachste Variante einer solchen Eingriffseinrichtung wäre die elektrostatische Aufladung der Außenseite des Stülpschlauchs und/oder des Stetigförderers, um eine Anziehungskraft auf das jeweils andere Bauteil zu erzeugen.

Der Stetigförderer selbst ist, wie vorstehend erwähnt, an dem Endoskopschaft 3 angebracht und weist einen radial inneren und äußeren Abschnitt auf. Hierbei sind mehrere Anordnungen des Stetigförderers bezüglich des Stülpschlauchs möglich. Die in diesem Ausführungsbeispiel beschriebene Anordnung beschreibt einen Stetigförderer, der mit der am Stülpschlauch befindlichen Eingriffseinrichtungskomponente lediglich an einem Abschnitt, nämlich dem radial äußeren Abschnitt des Stetigförderers, in Eingriff steht. D.h. der Stetigförderer befindet sich zumindest mit einem Abschnitt zwischen dem Endoskopschaft 3 und dem Stülpschlauch 2, während sich bei dem nachstehend beschriebenen vierten Ausführungsbeispiel lediglich ein Abschnitt des Stetigförderers zwischen dem Endoskopschaft 3 und den Stülpschlauch 2 und ein weiterer radial äußerer Abschnitt des Stetigförderers an der radial äußeren Seite des radial äußeren Abschnitts des Stülpschlauchs befindet, d.h. der Stetigförderer umgibt den Stülpschlauch.

Ebenso wie bei der Eingriffseinrichtung sind verschiedene Ausführungsformen für den Stetigförderer denkbar. Dieser kann durch ein endloses Fördermittel ausgebildet werden, beispielsweise einen Riemen, ein Band, vorzugsweise ein Kugelband, einen Faden oder eine Kette usw. Aber ebenso wäre ein Stetigförderer einer anderen Bauart anwendbar, solange der Eingriff mit der am Stülpschlauch befindlichen Eingriffseinrichtungskomponente im wesentlichen fortlaufend hergestellt werden kann.

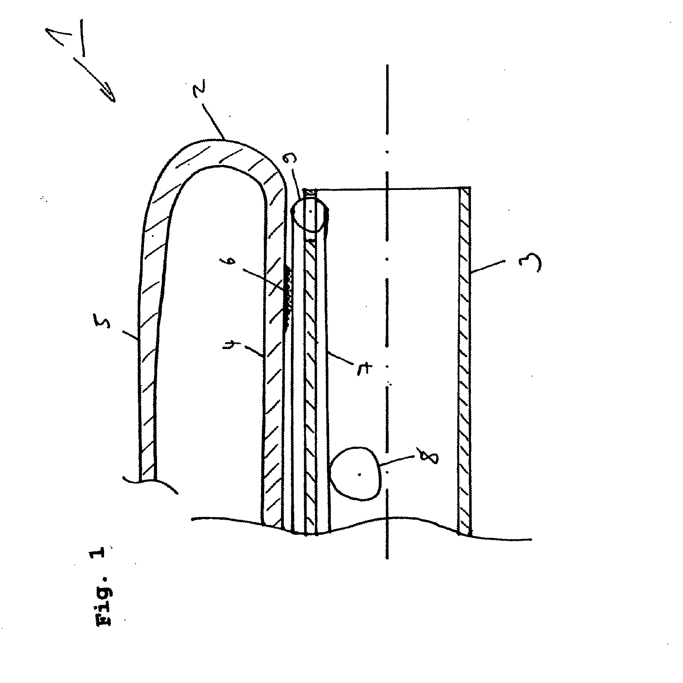

Bezüglich der vorstehend erwähnten Anordnung des Stetigförderers dieses Ausführungsbeispiels kann die Führung des Stetigförderers, der in diesem Ausführungsbeispiel als ein Band ausgebildet ist, beispielsweise durch an beiden Endabschnitten des Endoskopschafts 3 angebrachte Rollen 9 bewerkstelligt werden, die in Längsrichtung des Endoskopschafts voneinander beabstandet sind, wie in den Figuren 1 bis 3 ersichtlich ist. Für den Fall, dass die vorstehend erwähnten Rollen 9 an den beiden Endabschnitten des Endoskopschafts 3 angebracht sind, wovon Eines jeweils dem distalen Ende des Endoskopschafts und das Andere jeweils dem proximalen Ende des Endoskopschafts entspricht, wird der Eingriff zwischen dem Stetigförderer und der Stülpschlauchseitigen Komponente der Eingriffseinrichtung 6 je nach Bewegungsrichtung des Endoskopschafts bewirkt oder gelöst. Hierbei befindet sich die entsprechend der Eingriffseinrichtungskomponente des Stülpschlauchs 2 angepasste Eingriffseinrichtungskomponente des Stetigförderers 7 an der Außenseite des Stetigförderers bzw. in diesem Ausführungsbeispiel des Fließbands. Die den Stetigförderer 7 führenden Rollen 9 können hierbei derart an dem Endoskopschaft 3 angebracht sein, dass der Stetigförderer 7 sich gänzlich radial außerhalb des Endoskopschafts 3 befindet oder jeweils ein Abschnitt, der radial äußere Abschnitt, des Stetigförderers 7 radial außerhalb des Endoskopschafts 3 entlang dessen Längsrichtung verläuft, während der andere Abschnitt, der radial innere Abschnitt, des Stetigförderers 7 radial innerhalb des Endoskopschafts 3, beispielsweise innerhalb eines in dem Endoskopschaft ausgebildeten Kanals, verläuft, wie letzteres schematisch in der Fig. 1 gezeigt ist.

Ebenso wäre eine Führung des Stetigförderers 7 denkbar, die keine Rollen verwendet. So kann eine Nut an dem Außenumfang des Endoskopschafts 3 ausgebildet sein, die entlang des Endoskopschafts in dessen Längsrichtung ausgebildet ist und weiter in einem radial innerhalb des Endoskopschafts ausgebildeten Kanal verläuft, so dass eine durchgängige Nut ausgebildet wird. In diese kann der Stetigförderer eingesetzt bzw. eingebettet werden, wodurch die Führungsfunktion des Stetigförderers alleine von der vorstehend beschriebenen Nut anstelle der Rollen übernommen werden kann.

Durch den an dem Endoskopschaft 3 angebrachten Stetigförderer 7, der, wie vorstehend erwähnt, durch die Rollen 9 geführt wird oder in der umlaufenden Nut angeordnet ist, kann der Endoskopschaft 3 unabhängig von der Bewegung des Stülpschlauchs 2 bewegt werden, d.h. eine Relativbewegung zu dem Stülpschlauch 2 ausführen.

Die oben beschriebene Anordnung des Stetigförderers, aufgrund welcher der Endoskopschaft 3 relativ zu dem Stülpschlauch 2 bewegbar ist, kann auf unterschiedliche Arten zum Vorwärtsbewegen des Stülpschlauchs als auch des Endoskopschafts angetrieben werden. Dies kann zum einen durch einen unmittelbar auf den Endoskopschaft einwirkenden Antrieb und zum Anderen durch einen Stülpschlauchantrieb erfolgen, bei dem der Stetigförderer quasi über den Stülpschlauch angetrieben wird.

Gemäß dem ersten Ausführungsbeispiel der vorliegenden Erfindung wird das oben beschriebene Endoskop mit Ausstattung eines auf den Stülpschlauch unmittelbar einwirkenden Stülpschlauchantriebs beschrieben.

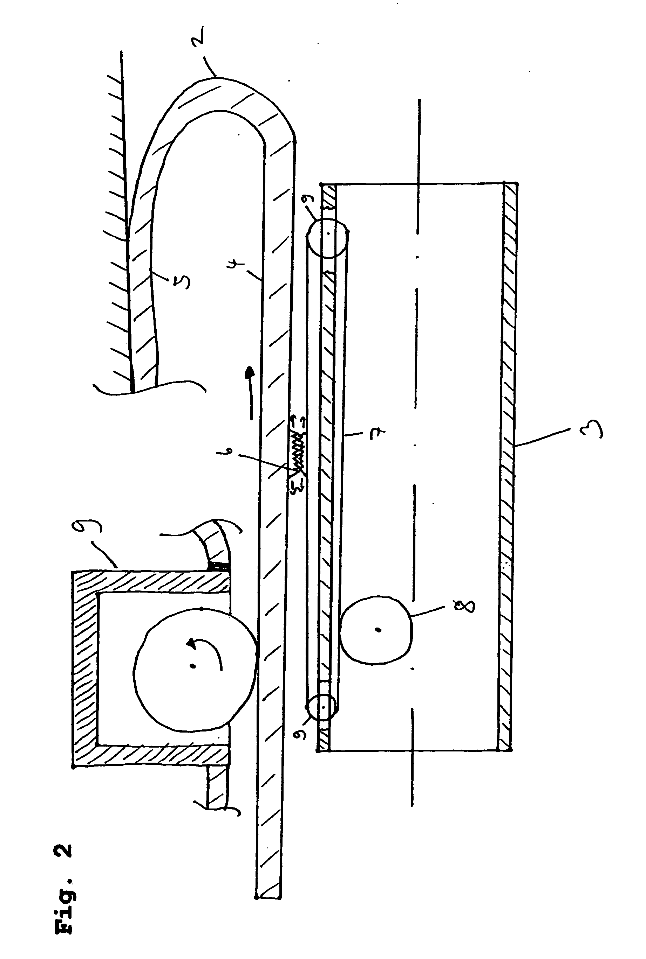

Im vorliegenden Fall wird unter Stülpschlauchantrieb das Antreiben des radial inneren Abschnitts 4 des Stülpschlauchs 2 mittels einer Antriebeinrichtung 9 verstanden, wie aus Figur 2 ersichtlich ist. Da aber erfindungsgemäß auch eine Relativbewegung des Endoskopschafts zu dem Stülpschlauch ermöglicht sein muss, sind weitere Ausgestaltungen des Endoskops erforderlich.

Durch Antreiben des radial inneren Abschnitts 4 des Stülpschlauchs 2 in die Einführrichtung (entsprechend dem Verlauf des Hohlkanals, beispielsweise des Darms) bei gleichzeitiger Ruhelage des radial äußeren Abschnitts 5 des Stülpschlauchs, der an der Wandung des Hohlkanals, beispielsweise der Darmwand, anliegt, bewegt sich der Stülpschlauch 2 quasi "abrollend" voran. Dadurch passt sich der Stülpschlauch 2 den zu durchlaufenden Kanal, beispielsweise dem Darm, an, ohne diesen bei der Vorwärtsbewegung auszubauchen. Mittels des Eingriffszustands zwischen dem an dem Endoskopschaft 3 befindlichen Stetigförderer 7 und dem radial inneren Abschnitt 4 des Stülpschlauchs 2 wird der Stetigförderer 7, in diesem Fall das umlaufende Endlosband vorangetrieben, ohne dass ein Vorwärtsantrieb auf den Endoskopschaft 3 übertragen wird. Das Band läuft sozusagen leer. Um nunmehr eine Kraftübertragung auf den Endoskopschaft 3 zu erreichen, ist hierfür eine Art Ausgleichseinrichtung 8 (oder eine Synchronisationseinrichtung) an dem Endoskopschaft 3 angebracht, die mit dem Stetigförderer 7 entweder über eine Kopplung mit einer der Rollen 9 oder durch Kontakt mit dem Endlosband in Eingriff steht.

Die Ausgleichseinreichung 8 besteht demzufolge aus einem Getriebemechanismus oder einem Ausgleichsmotor, wodurch die Vorwärtsgeschwindigkeit des Endlosbands derart untersetzt wird, dass sich die Vorwärtsgeschwindigkeit des Endoskopschafts der Vorwärtsgeschwindigkeit des vorderen Stülpabschnitts des Stülpschlauchs angleicht. Die Verwendung eines Ausgleichsmotor ist daher zudem gegenüber einem bloßen Getriebe in so fern vorteilhaft, als dass hierdurch die Bewegung des Stetigförderers 7 relativ zu der Bewegung des Stülpschlauchs gesteuert (voraus-/nacheilend) werden kann.

Ein Blockieren des Stetigförderers 7 durch die entsprechendes Anhalten der Ausgleichseinrichtung 8 bewirkt, dass sich der Endoskopschaft 3 bei Bewegung des Stülpschlauchs 2 mitbewegt, jedoch schneller, als sich der Stülpschlauch 2, bzw. dessen vorderer Umstülpabschnitt in dem zu durchlaufenden Kanal voranbewegen kann. Daher ist es erforderlich, die Differenzgeschwindigkeit zwischen Stülpschlauch 2 und Endoskopschaft 3 durch die Ausgleichseinrichtung 8 so zu kompensieren bzw. auszugleichen, dass der Endoskopschaft 2 mit gleicher (oder auch geringerer) Geschwindigkeit voranbewegt wird, wie der Stülpschlauch 2 voranschreitet. Daher bringt die Ausgleichseinrichtung 8 eine relative Bewegung auf den Stetigförderer 7 auf, so dass sich der Endoskopschaft 3 bei Stillstand des radial inneren Abschnitts 4 des Stülpschlauchs 2 gegen die Einführrichtung bewegen würde. Da sich aber der Stülpschlauch 2 voranbewegt und eine Relativbewegung des Endoskopschafts 3 gegenüber dem Stülpschlauch 2 mittels der Ausgleichsvorrichtung 8 erzeugt werden kann, kann die Lage bzw. die Geschwindigkeit des Endoskopschafts 3 gesteuert werden. Somit kann ein Vorauseilen des Endoskopschafts 3 mittels geeigneter Steuerung der Ausgleichseinrichtung 8 verhindert werden, so dass der Stülpschlauch 2 fortwährend dem Endoskopschaft 3 vorauseilt bzw. sich der Stülpschlauch und der Endoskopschaft mit gleicher Geschwindigkeit voranbewegen.

Auch bei Stillstand des Stülpschlauchs 2 kann die Lage des Endoskopschafts 3 mittels der Ausgleichseinrichtung 8 beliebig geändert werden. Weiter werden durch solch einen Aufbau des Endoskops die in dem Stand der Technik verwendeten und auch benötigten Anschlagstücke überflüssig, wodurch kein Leistungsabfall aufgrund von Reibung zwischen diesen und dem Stülpschlauch Zustande kommt.

Vorliegend wurde ein an dem Endoskopschaft angebrachter Stetigförderer oder eine an dem Stülpschlauch ausgebildete Eingriffseinrichtung beschrieben. Jedoch können zwei, drei oder mehrere Stetigförderer über den Umfang an dem Endoskopschaft in gleichem Umfangsabstand zueinander beabstandet angeordnet sein. Entsprechend können zwei, drei oder mehrere Eingriffseinrichtungen über den Innenumfang des Stülpschlauchs in gleichem Umfangsabstand zueinander beabstandet angeordnet sein.

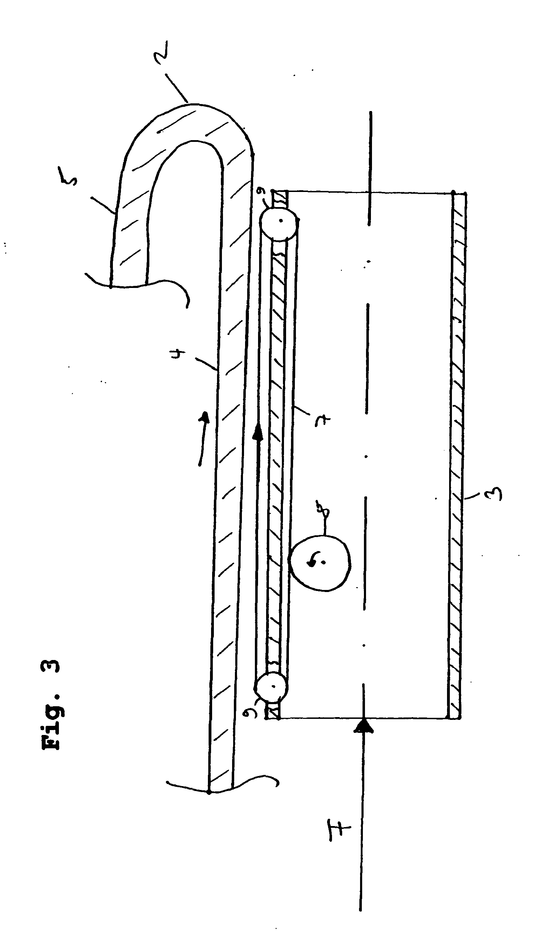

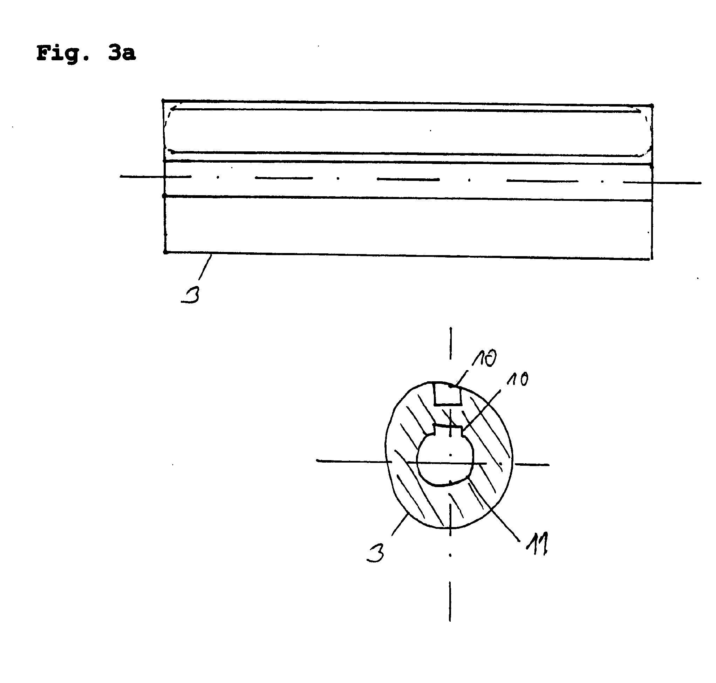

Gemäß einem zweiten Ausführungsbeispiel der vorliegenden Erfindung wird das Endoskop des ersten Ausführungsbeispiels alternativ mit Ausstattung eines unmittelbar auf den Endoskopschaft einwirkenden Antriebs beschrieben, wie aus Figur 3 ersichtlich ist.

Unter dieser Art Antrieb wird üblicherweise verstanden, dass lediglich der Endoskopschaft mittels einer Antriebseinrichtung direkt angetrieben wird. Angewandt auf das nun folgende zweite Ausführungsbeispiel der vorliegenden Erfindung weist der Endoskopschaft 3 eine Antriebseinrichtung (nicht weiter dargestellt) auf, die diesen in dem Stülpschlauch 2 voranbewegt. Durch den über die Eingriffseinrichtung 6 mit dem Stülpschlauch in Eingriff befindlichen Stetigförderer 7 wird die Bewegung des Endoskopschafts 3 auf den radial inneren Abschnitt 4 des Stülpschlauchs 2 übertragen, der sich dadurch wie vorstehend beschrieben in dem Kanal, beispielsweise dem Darm, voranbewegt. Da der Endoskopschaft 3 schneller voranschreitet als der Stülpschlauch 2, wie vorstehend beschrieben, ist der Endoskopschaft 3 ebenfalls mit der Ausgleichseinrichtung 8 ausgestattet, die den Stetigförderer 7 derart bewegt (antreibt), dass der Stülpschlauch 2 seine quasi "Abrollbewegung" schneller durchführt, d.h. sich schneller in die Einführrichtung des Endoskopschafts bewegt. Dadurch wird ein Vorauseilen des Endoskopschafts 3 gegenüber dem Stülpschlauch 2 verhindert.

Der Unterschied dieses zweiten Ausführungsbeispiel zu dem vorigen ersten Ausführungsbeispiel der Erfindung besteht demzufolge darin, dass hier die Geschwindigkeit des Endoskopschafts vorgegeben wird bzw. die Vorwärtsbewegung/Einführbewegung des Endoskopschafts und anhand der Ausgleichseinrichtung 8 die Geschwindigkeit des Stülpschlauchs eingestellt wird, während in dem vorigen Ausführungsbeispiel die Geschwindigkeit des Stülpschlauchs 2 vorgegeben wird und anhand der Ausgleichseinrichtung 8 die Geschwindigkeit des Endoskopschafts eingestellt wird.

Gemäß einem dritten Ausführungsbeispiel der vorliegenden Erfindung ist der Stetigförderer 7, wie vorstehend bei dem ersten Ausführungsbeispiel der vorliegenden Erfindung erwähnt, prinzipiell auf eine den Stülpschlauch unmittelbar umgebende Weise angeordnet bzw. ausgeführt.

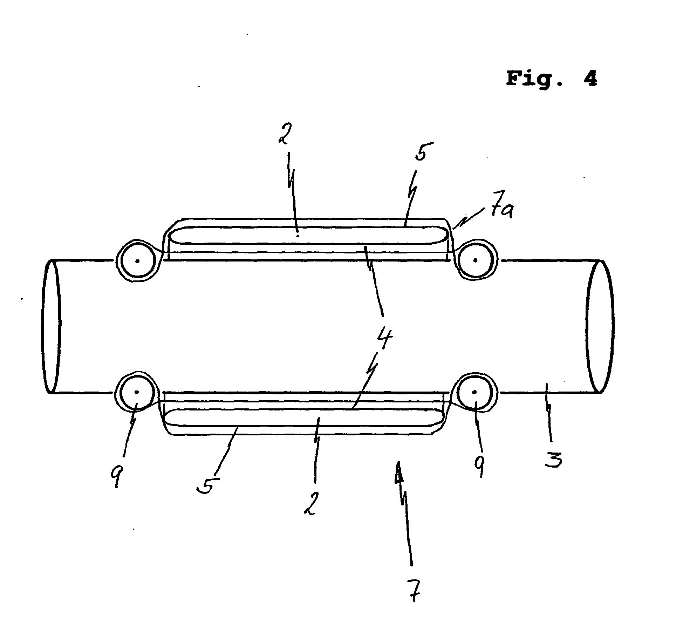

Konkret weist der Endoskopschaft 3 gemäß dem dritten Ausführungsbeispiel der Erfindung zumindest ein umlaufendes Endlosband oder Faden 7a auf, das an einer proximalen und distalen Rolle 9 geführt ist, welche in Längsrichtungsabstand des Endoskops am Endoskopschaft 3 angeordnet sind. Wie hierbei aus der Fig. 4 zu entnehmen ist, sind die Rollen 9 ähnlich zu dem vorstehend beschriebenen ersten und zweiten Ausführungsbeispiel der Erfindung so am Endoskopschaft 3 gelagert, dass diese geringfügig über die Mantelfläche des Schafts 3 vorragen.

Im Unterschied zu dem ersten und zweiten Ausführungsbeispiel verläuft das Endlosband 7a gemäß dem dritten Ausführungsbeispiel der Erfindung nicht ausschließlich zwischen dem Endoskopschaft 3 und dem radial inneren Abschnitt 4 des Stülpschlauchs 2, sondern der außerhalb des Endoskopschafts 3 befindliche Teil des Endlosbands oder Fadens 7a umspannt den gesamten Stülpschlauch 7a und ist somit mit dem radial äußeren Abschnitt 5 des Stülpschlauchs 2 in Wirkeingriff.

Konkret ist das Endlosband oder Faden 7a gemäß der Fig. 4 an den zwei längsbeabstandeten Rollen 9 geführt, welche am Endoskopschaft 3 gelagert sind. Zwischen den beiden Rollen 9 ist der Doppelstülpschlauch 2 angeordnet. Dieser weist zumindest eine Längsnut (nicht weiter dargestellt) auf, die sich an der Außenseite des Stülpschlauchs 2 erstreckt und in Längsrichtung des Stülpschlauchs 2 vollständig umlaufend ist. Die Nut bildet eine Längshinterschneidung beispielsweise indem die Nut im Querschnitt T-förmig ausgestaltet ist und dient zur Aufnahme des Endlosbands bzw. des Fadens 7a. In der Fig. 4 ist der Faden 7a im Abstand zum Stülpschlauch 2 dargestellt. Dies ist jedoch nur eine Prinzipdarstellung. In Wirklichkeit liegt der Faden 7a innerhalb der im Stülpschlauch 2 ausgebildeten Nut und befindet sich damit in enger Anlage mit dem Stülpschlauch. Aufgrund der Flexibilität des Schlauchmaterials kann dabei der Faden 7a an beiden Umstülpabschnitten des Schlauchs 2 aus den Nuten treten und um die Rollen 9 geführt werden.

Bei dieser Konstruktion kann der Stülpschlauch zwangsläufig nicht über die proximalen oder distalen Rollen 9 des Stetigförderers voreilen, da dessen proximaler oder distaler Umstülpabschnitt vom jeweils äußeren Abschnitt des Endlosbands oder Fadens 7a zurückgehalten wird. Der Stetigförderer 7 gemäß dem dritten Ausführungsbeispiel der Erfindung übernimmt demnach auch die Funktion eines vorderen und hinteren Anschlagstücks, wie sie aus dem eingangs beschriebenen Stand der Technik bekannt sind. Darüber hinaus bewirkt die Nutenführung des Bands oder Fadens 7a, dass sich dieser bei Abkrümmen des Endoskopschafts nicht vom Endoskopschlauch abhebt.

Die Funktionsweise hinsichtlich des Antriebs des Endoskopschafts 3 und des Stülpschlauchs 2 ist die Gleiche wie die des ersten und zweiten Ausführungsbeispiels, sodass an dieser Stelle auf die vorstehenden Beschreibungstextstelen verwiesen werden kann.

Abschließend sei darauf hingewiesen, dass das Endlosband oder umlaufender Endlosfaden vorzugsweise so um die proximalen und distalen Rollen 9 geführt ist, dass sie diese jeweils zumindest einmal vollständig umschlingen, wie dies in der Fig. 4 dargestellt ist. Dies Wicklungsart hat den Vorteil, dass der proximale und distale Umstülpabschnitt des Stülpschlauchs 2 in Richtung zur Mantelfläche des Endoskopschafts 3 gezogen wird, sodass ein Aufspreizen des Stülpschlauchs 2 insbesondere im distalen Bereich verhindert werden kann. Ferner ist es vorteilhaft, den Stülpschlauch 2 mit den Längsnuten zur Aufnahme des Endlosbands oder Fadens gemäß vorstehender Beschreibung zu versehen, sodass eine bessere Kraftübertragung zwischen Stülpschlauch und Stetigförderer 7 erfolgt und darüber hinaus die Verletzungsgefahr durch vorragende Teile insbesondere am äußeren Abschnitt 5 des Stülpschlauchs 2 verringert wird.

Alternativ zu der dargestellten Führung des Fadens oder Bands 7a um die Rollen 9 sind natürlich auch andere Möglichkeiten für das Ineingriffkommen zwischen Faden und Rolle denkbar. So könnten die Rollen eine Art Klemmnut in Umfangsrichtung aufweisen, in die der Faden eingeführt ist.

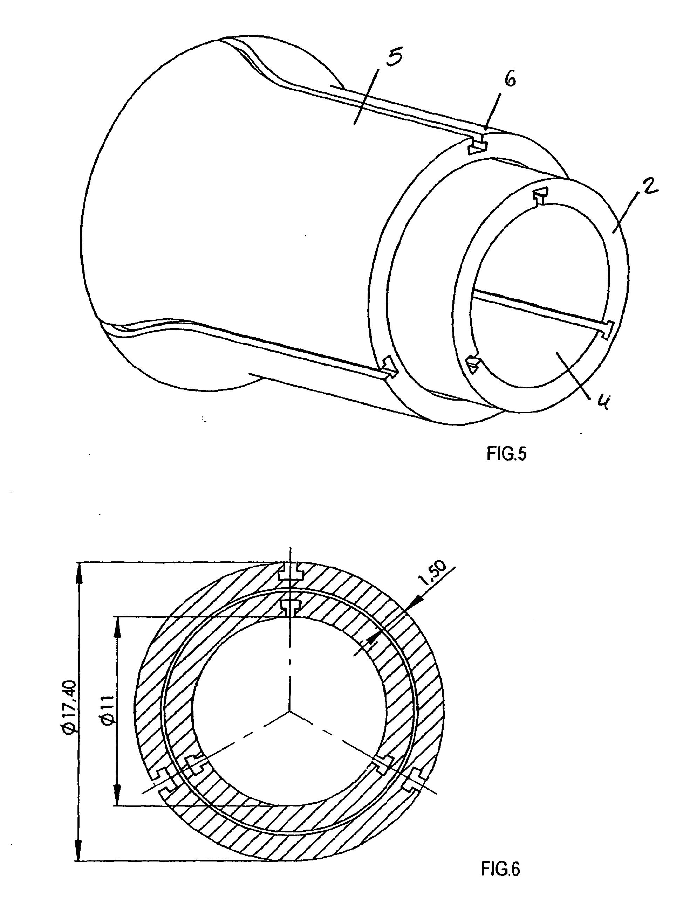

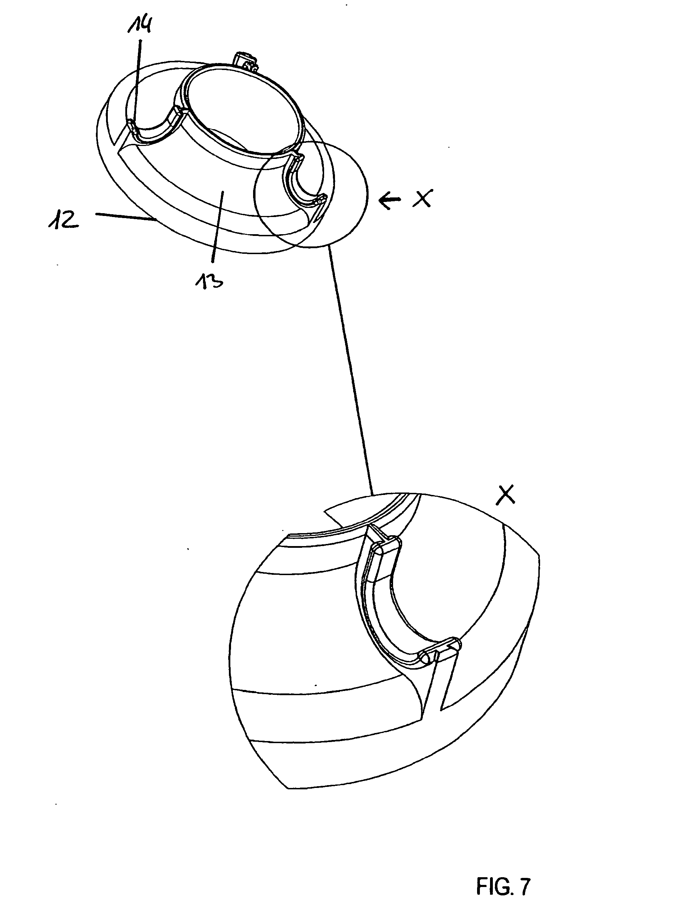

Schließlich wird nachfolgend ein durch die Erfindung nicht abgedecktes erstes Beispiel anhand der Fig. 5 bis 8 beschrieben.

Gemäß den Figuren 5 bis 8 besteht das Endoskop des ersten Beispiels im Wesentlichen aus einem Endoskopschaft 3 sowie einem diesen zumindest partiell umgebenden Stülpschlauch 2. Der Stülpschlauch 2 weist einen radial inneren Stülpschlauchabschnitt auf, der zumindest an einem distalen Ende des Endoskopschafts 3 zu einem radial äußeren Stülpschlauchabschnitt umgestülpt ist. Der Stülpschlauch 2 weist auf einer äußeren Schlauchseite zumindest eine Längsführung auf. Diese besteht aus einem in Längsrichtung des Stülpschlauchs 2 sich erstreckenden Schlitz oder einer Nut, welche in ihrem Nutengrund im Querschnitt eine T-Hinterschneidung ausbildet. Alternativ hierzu könnte die Längsnut auch in Form einer Schwalbenschwanzführung oder dergleichen Hinterschneidung ausgebildet sein.

Vorliegend sind drei in gleichem Umfangsabstand zueinander angeordnete Längsnuten ausgebildet. Alternativ hierzu könnten aber auch nur eine, zwei oder mehr als die drei gezeigten Nuten vorgesehen sein.

Wie insbesondere aus der Fig. 5 zu entnehmen ist, sind die Nuten auf jener Seite des Stülpschlauchs angeordnet, welche nach Durchlaufen des distalen Umstülpabschnitts bzw. des radial inneren Abschnitts auf der Außenseite des äußeren Stülpschlauchabschnitts bzw. des radial äußeren Abschnitts zu liegen kommt.

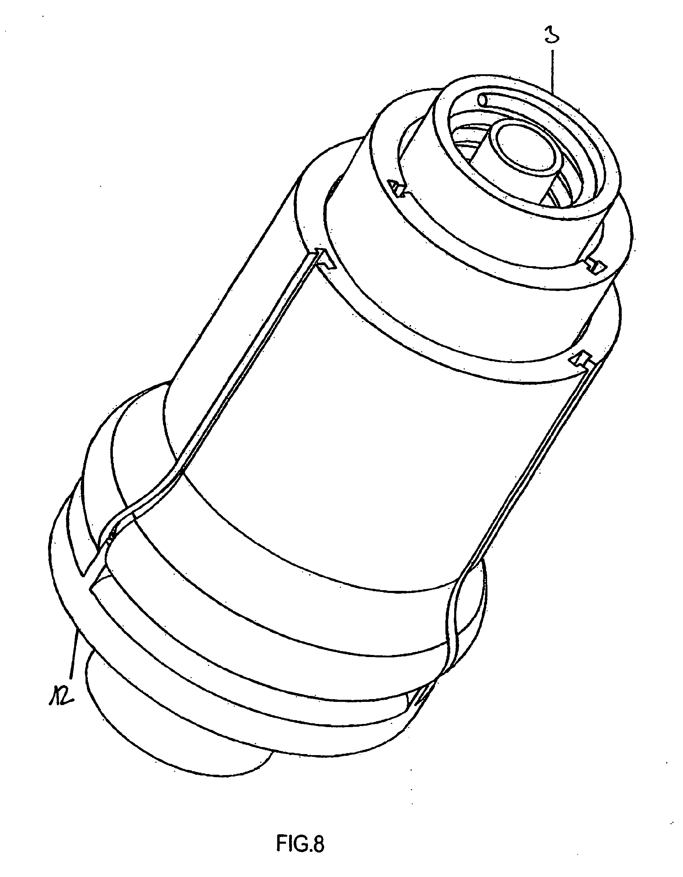

Der in der Fig. 8 schematisch dargestellte Endoskopschaft 3 besteht aus einem vorzugsweise mittels einer Spiralfeder oder einem Fadengespinst laminierten Mantel, in welchem ein Arbeitskanal ausgebildet ist. An einem distalen Endabschnitt des Endoskopschafts 3 ist ein radialer Vorsprung 12 ausgebildet, welcher eine dem Stülpschlauch zugewandte sowie vorzugsweise dem distalen Umstülpabschnitt des Stülpschlauchs angepasste Gleitfläche 13 ausbildet. In dieser Gleitfläche 13 sind eine Anzahl von Gleitschienen 14 ausgeformt, deren Querschnitt dem Querschnitt der im Stülpschlauch 2 ausgebildeten Nuten entspricht. Des weiteren sind die Gleitschienen 14 entsprechend den Nuten im Stülpschlauch 2 in Umfangsrichtung beabstandet.

Beim Montieren des Stülpschlauchs 2 wird dieser in vorerst ungestülptem Zustand auf den Endoskopschaft 3 derart aufgezogen, dass die am distalen Endabschnitt des Endoskopschafts 3 vorgesehenen Gleitschienen 14, welche sich in Bewegungslängsrichtung des Stülpschlauchs 2 erstrecken und entsprechend der Gleitfläche 13 bzw. der Form des distalen Umstülpabschnitts des Stülpschlauchs im Wesentlichen U-förmig angepasst sind, in die zugehörigen Nuten eingefädelt werden. Hierdurch wird der Stülpschlauch am distalen Endabschnitt des Endoskopschafts zwangsläufig umgestülpt, wodurch sich der äußere Umstülpabschnitt bildet, welcher sich in Richtung zum proximalen Ende (nicht gezeigt) des Endoskopschafts 3 zurückbewegt.

Der am distalen Endabschnitt des Endoskopschafts 3 angeordnete radiale Vorsprung 12, bzw. die daran ausgebildeten, entsprechend geformten Gleitschienen 14 kommen demzufolge mit den im Stülpschlauch 2 ausgebildeten Nuten in Gleiteingriff, wobei die durch die T-Form entstehenden Hinterschneidungen am Nutengrund mit den entsprechenden T-förmigen Vorsprüngen der Gleitschienen 14 in Führungseingriff kommen und somit ein Herausziehen der Gleitschienen 14 aus der Nut des Stülpschlauchs 2 verhindern. Auf dieses Weise kann über den radialen Vorsprung des Endoskopschafts 3 eine den Stülpschlauch 2 in Bewegungslängsrichtung wirkende Zug- oder Streckkraft ausgeübt werden.

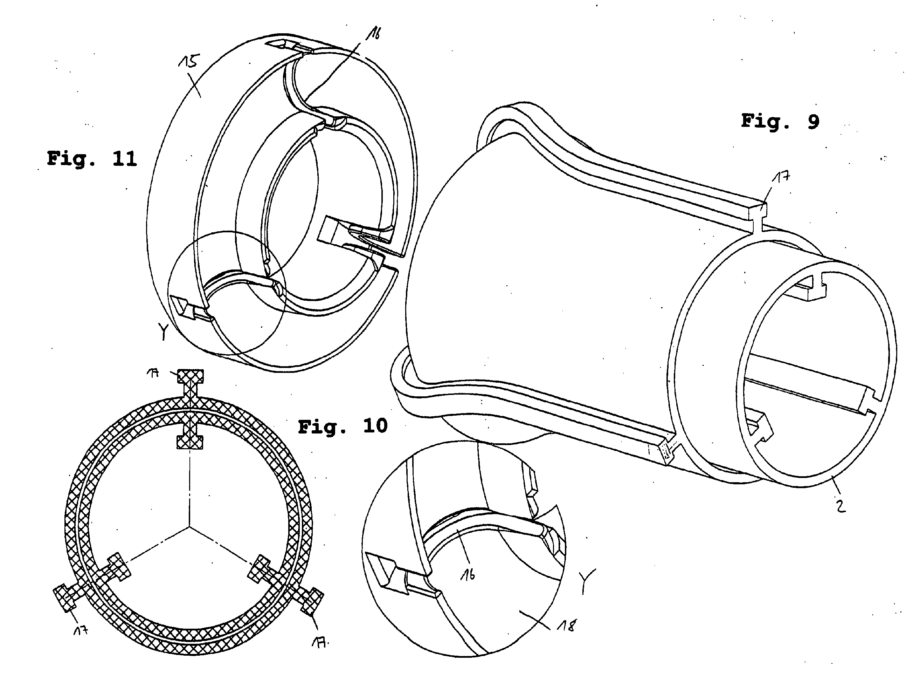

In den Figuren 9 bis 11 ist ein zum ersten Beispiel alternatives, durch die Erfindung nicht abgedecktes zweites Beispiel dargestellt. Das Konstruktionsprinzip des zweiten Beispiels entspricht im Wesentlichen jenem des vorstehend beschriebenen ersten Beispiels, wobei indessen die Gleitschienenanordnung bzw. die Nutenanordnung jeweils am Stülpschlauch bzw. am distal angeordneten Vorsprung des Endoskopschafts untereinander vertauscht sind. In anderen Worten ausgedrückt hat gemäß dem zweiten Beispiel der Stülpschlauch 2 längs sich erstreckende, vorzugsweise T-förmige (oder andersartig gestaltete wie Schwalbenschwanz-förmige, L-förmige etc.) Gleitschienen 17, welche auf einer Mantelseite radial vorstehen.

Auch in diesem Fall ist die Seite, auf welcher die Gleitschienen 17 am Stülpschlauch 2 angeordnet sind, so gewählt, dass sich diese im vorderen Umstülpabschnitt sowie am äußeren Umstülpabschnitt des Stülpschlauchs 2 nach außen vorstehen.

In der Fig. 9 sind die am Stülpschlauch vorgesehenen Gleitschienen 17 im Querschnitt T-förmig ausgestaltet und, wie dies in der Fig. 10 dargestellt ist, in Umfangsrichtung gleichmäßig voneinander beabstandet. Vorzugsweise sind drei Gleitschienen 17 vorgesehen, wobei die Zahl auch auf zwei oder mehr als drei Gleitschienen 17 verändert werden kann. Alternativ zu der T-Form kann jede Gleitschiene auch Schwalbenschwanzförmig oder andersartig ausgestaltet sein, solange eine zur Führung des Stülpschlauchs geeignete Hinterschneidung ausgebildet ist.

Des Weiteren ist jede Gleitschiene 17 in der anliegenden Fig. 9 in Längsrichtung des Stülpschlauchs 2 durchgehend dargestellt. Indessen sei darauf hingewiesen, dass zur Erzielung eines möglichst engen Umstülpabschnitts und darüber hinaus einer hohen Flexibilität des gesamten Endoskops die Gleitschienen 17 auch in eine Vielzahl von Gleitschienenstücken unterbrochen sein können, wodurch Soll-Biegestellen zur Reduzierung der Biegesteifigkeit des Stülpschlauchs definiert werden.

Die Fig. 11 zeigt den radialen Vorsprung 15, wie er am distalen Endabschnitt des Endoskopschafts 3 ausgebildet bzw. angeordnet ist. Auch bei diesem Vorsprung 15 ist eine dem Stülpschlauch 2 zugewandte Gleitfläche 18 ausgeformt, welche im Wesentlichen der Form des vorderen Umstülpabschnitts angenähert ist und daher eine im Wesentlichen wannen- oder rinnenförmige Kontur annimmt. Diese Kontur entspricht im übrigen auch jener des zweiten Beispiels.

Entgegen dem ersten Beispiel sind jedoch auf dieser Gleitfläche 18 in Umfangsrichtung gleichmäßig beabstandete Nuten ausgeformt, welche sich in Gleitrichtung des Stülpschlauchs 2, das heißt, die rinnenförmige Gleitfläche durchquerend erstrecken und deren Nutengrund jeweils mit T-förmigen oder entsprechend den Gleitschienen 17 ausgebildeten Hinterschneidungen versehen ist. Die Form sowie die Abstände der im radialen Vorsprung vorgesehenen Nuten ist demnach derart, dass bei Aufziehen des Stülpschlauchs 2 auf den Endoskopschaft 3 die daran vorgesehenen Gleitschienen 17 in die Nuten 16 des radialen Vorsprungs eingefädelt werden können, wodurch der Stülpschlauch 2 im Bereich des radialen Vorsprungs 15 zwangsläufig zu dem äußeren Stülpschlauchabschnitt umgestülpt wird. Darüber hinaus kann über den radialen Vorsprung 15 und die darin ausgebildeten Nuten in Übereinstimmung mit dem vorstehend beschriebenen ersten Beispiel eine Zug- bzw. Spannkraft auf den Stülpschlauch ausgeübt werden, welche den Stülpschlauch 2 in Längsrichtung des Endoskopschafts 3 streckt.

Ein derart gestaltetes Endoskop lässt sich wie folgt betätigen:
Ohne Stülpschlauchantrieb (passives System):
   Ein Endoskop mit dem beschriebenen prinzipiellen Aufbau gemäß dem ersten und zweiten Beispiel lässt sich durchaus per Hand in einen kanalartigen Hohlraum wie bspw. den Darm eines Patienten einführen. In diesem Fall wird eine Vorschubkraft per Hand unmittelbar auf den Endoskopschaft an dessen proximalem Endabschnitt aufgebracht, wodurch der Endoskopschaft 3 in den Darm des Patienten vorwärts getrieben wird. Die hohe Flexibilität des Endoskopschafts sowie die Anordnung weiteren Vorrichtung wie bspw. ein Deflecting, welches am distalen Ende des Endoskopschafts an diesem angeordnet ist,
   erlauben es, den Endoskopschaft 3 auch um Biegungen des Darmsystems herumzuführen, ohne dass der Endoskopschaft 3 ein Aufweiten der Darmwindungen verursacht.

Bei einem derartigen Vortrieb wird der Stülpschlauch quasi passiv angetrieben, indem dessen äußerer Umstülpabschnitt an der Darmwandung quasi haftet und somit den inneren Stülpschlauchabschnitt mit einer bezüglich der Bewegungsgeschwindigkeit des Endoskopschafts doppelten Geschwindigkeit in Richtung des distalen radialen Vorsprungs des Endoskopschafts zieht. Diese Zugkraft wird dabei über den radialen Vorsprung und den daran ausgebildeten Leitschienen oder Nuten gemäß dem vorstehend beschriebenen ersten oder zweiten Beispiel auf den distalen Umstülpabschnitt bzw. die daran ausgebildeten Nuten bzw. Gleitschienen des Stülpschlauchs übertragen, wobei der Stülpschlauch längs der Gleitfläche des radialen Vorsprungs abgleitet. Aufgrund der Längsführung am Stülpschlauch 2 sowie am radialen Vorsprung des Endoskopschafts 3 ist die Gleitreibung, welche zwangsläufig bei der Übertragung der Zugkräfte auf den vorderen Umstülpabschnitt des Stülpschlauchs entstehen sehr gering, so dass die Gesamtvorschubskraft, welche auf den Endoskopschaft für dessen Vortrieb in den Darm aufgebracht werden muss dementsprechend gering sind.

Mit zusätzlichem Stülpschlauchantrieb (aktives System):

Alternativ zu der vorstehend beschriebenen Antriebsweise des Endoskops ist es natürlich auch möglich, das erfindungsgemäße Endoskop mit einem Antriebsmechanismus für einen motorischen Vorschub auszurüsten. In diesem Fall würde eine motorische Antriebskraft auf den Stülpschlauch 2 aufgebracht werden, welche diesen in Längsrichtung des Endoskopschafts 3 bewegt. Dieser Antriebsmechanismus ist derart gestaltet, dass zumindest ein Teil der Antriebskraft vorzugsweise auf den Endoskopschaft 3 selbst aufgebracht wird, welcher eine Vorwärtsbewegung des Endoskopschafts 3 bewirkt. Zur Erreichung dieser Antriebsbewegung könnten bspw. zwei Antriebe vorgesehen sein, welche in deren Bewegungsgeschwindigkeit aufeinander abgestimmt sind und jeweils den Endoskopschaft 3, bzw. den Endoskopschlauch (Stülpschlauch 2) antreiben. Derartige Antriebseinrichtungen sind aus dem Stand der Technik bspw. gemäß der eingangs genannten Druckschriften bekannt, so dass an dieser Stelle auf die entsprechenden Druckschriften verwiesen werden kann.

Im Fall eines derartigen aktiven Systems d. h. eines Endoskops, bei welchem der Stülpschlauch 2 aktiv mittels einer Antriebsvorrichtung angetrieben wird, wird ein Teil der Antriebskraft, welcher auf den Stülpschlauch 2 aufgebracht wird über den distalen radialen Vorsprung indirekt auf den Endoskopschaft 3 für dessen Vortrieb übertragen. Zusätzlich wird der Endoskopschaft 3 selbst durch den Antriebsmechanismus unmittelbar vorwärtsbewegt, wodurch der Endoskopschaft gegebenenfalls dem distalen Umstülpabschnitt des Stülpschlauchs vorauseilen kann. Durch die Ausbildung der Längsführung der vorstehend beschriebenen Nuten-Führungsschienen-Kombination am Stülpschlauch sowie zumindest am distalen radialen Vorsprung wird jedoch der Stülpschlauch 2 bzw. dessen distaler Umstülpabschnitt mit dem Endoskopschaft quasi mitgezogen, so dass ein Aufschuppen oder Zurückbleiben des Stülpschlauchs wirksam verhindert wird. Da darüber hinaus durch die Ausbildung der erfindungsgemäßen Führungseinrichtung als Längsführung die Gleitreibung gering gehalten werden kann, können die Antriebskräfte für ein vorwärtsbewegen des Endoskops entsprechend klein eingestellt sein, so dass ein Ausknicken des Endoskopschafts, der vorzugsweise aus einem EPTFE Material besteht weitgehend verhinderbar ist.

Weiter können die Nut als auch die Gleitschienen mit einer einlaminierten Verstärkung versehen sein, um ein ungewolltes Lösen eines Eingriffszustands zwischen den jeweiligen Längsführungseinrichtung zu vermeiden. Die Verstärkung kann jeglicher Art sein, solange dadurch der Eingriffszustand zwischen Schiene/Nut verstärkt wird. Vorzugsweise werden ein in die Schiene und/oder in die Nut eingearbeiteter Draht, vorzugsweise ein Metalldraht, verwendet. Somit kann bei Auftreten einer in Eingriffslösungsrichtung wirkenden Kraft das Aufrechterhalten des Eingriffszustands gesichert werden.

Abschließend sei noch darauf hingewiesen, dass die vorstehend beschriebene Längsführung bestehend aus Führungsschiene sowie entsprechend gestaltete Nut mit Hinterschneidung keinesfalls nur im Bereich des distalen radial sich erstreckenden Vorsprungs vorgesehen sein muss, sondern sich stattdessen oder in Ergänzung hierzu auch längs des Endoskopschafts an dessen äußerer Mantelfläche ausgebildet sein kann. Das heißt, dass entsprechend gestaltetete Führungsschienen oder Nuten an der äußeren Mantelfläche des Endoskopschafts gleichsam zu den vorstehend beschriebenen Nuten oder Führungsschienen am distalen radialen Vorsprung ausgebildet sind, in denen der Endoskopschlauch im Bereich seines inneren Schlauchabschnitts eingefädelt, bzw. eingeführt ist. Diese Art der Längsführung wäre auch ohne die Ausbildung entsprechender Führungsschienen oder Nuten am distalen Vorsprung des Endoskopschafts möglich, wobei jedoch mit erhöhten Reibungskräften am distalen Austrittsbereich des Stülpschlauchs, d. h., in jenem Bereich, an welchem die Führungsschienen oder Nuten am distalen Endabschnitt des Endoskopschafts aus den entsprechenden Gegenstücken heraustreten, zu rechnen ist. Um dies wirkungsvoll zu vermeiden ist auch eine Kombination eines derart ausgestalteten Endoskopschafts mit einem radialen Vorsprung gemäß dem vorstehend beschrieben dritten Ausführungsbeispiel oder ersten Beispiel denkbar.

## Patentansprüche

1. Endoskop mit einem einen radial äußeren Abschnitt (5) und einen radial inneren Abschnitt (4) aufweisenden Stülpschlauch (2) und einem zumindest teilweise im Stülpschlauch befindlichen Endoskopschaft (3), wobei Längsführungseinrichtungen (6, 7) jeweils an dem Endoskopschaft (3) und dem Stülpschlauch (2) ausgebildet sind, die miteinander in Eingriff stehen, wobei
die an dem Endoskopschaft ausgebildete Längsführungseinrichtung ein Stetigförderer ist,
**dadurch gekennzeichnet, dass**
der Stetigförderer als Riemen oder Band ausgebildet ist und in Längsrichtung entlang der Außenseite des Endoskopschafts geführt ist.

2. Endoskop gemäß Anspruch 1, **dadurch gekennzeichnet, dass** die an dem Stülpschlauch (2) ausgebildete Längsführungseinrichtung (6) eine Eingriffseinrichtung ist.

3. Endoskop gemäß Anspruch 2, **dadurch gekennzeichnet, dass** eine Antriebseinrichtung (9) den radial inneren Abschnitt (4) in dessen Längsrichtung bei gleichzeitiger Ruhelage des radial äußeren Abschnitts (5) vorantreibt und eine an dem Endoskopschaft (3) befindliche Ausgleichseinrichtung (8) den mit der Eingriffseinrichtung (3) in Eingriff stehenden Stetigförderer (7) antreibt, wodurch eine Relativbewegung des Endoskopschafts (3) gegenüber dem Stülpschlauch (2) steuerbar ist.

4. Endoskop gemäß Anspruch 2, **dadurch gekennzeichnet, dass** eine Antriebseinrichtung (9) den Endoskopschaft (3) in dessen Längsrichtung bewegt und eine an dem Endoskopschaft (3) befindliche Ausgleichseinrichtung (8) den mit der Eingriffseinrichtung (6) in Eingriff stehenden Stetigförderer (7) antreibt, wodurch eine Relativbewegung des radial inneren Abschnitts (4) bei gleichzeitiger Ruhelage des radial äußeren Abschnitts (5) zu dem Endoskopschaft (3) steuerbar ist.

5. Endoskop gemäß Anspruch 2, **dadurch gekennzeichnet, dass** der Stetigförderer den Stülpschlauch umgibt und vorzugsweise von an den beiden Enden des Endoskopschafts angebrachten Rollen geführt wird, die zumindest teilweise, vorzugsweise einmal oder mehrmals von dem Stetigförderer umschlungen werden.

6. Endoskop gemäß Anspruch 5, **dadurch gekennzeichnet, dass** der Stetigförderer weiter durch eine an dem Außenumfang des Endoskopschafts in dessen Längsrichtung verlaufende Nut geführt wird.

7. Endoskop gemäß Anspruch 5 oder 6, **dadurch gekennzeichnet, dass** eine Antriebseinrichtung entweder den Stetigförderer antreibt oder den Endoskopschaft in dessen Längsrichtung voranbewegt.

8. Endoskop gemäß einem der Ansprüche 3 bis 4, **dadurch gekennzeichnet, dass** der Stetigförderer (7) durch an dem Endoskopschaft (3) angebrachte Umlenkrollen (9) geführt wird, wobei ein radial äußerer Abschnitt des Stetigförderers (7) mit der am radial inneren Abschnitt des Stülpschlauchs befindlichen Eingriffseinrichtung (6) im Eingriff steht und ein radial innerer Abschnitt des Stetigförderers (7) mit der an dem Endoskopschaft (3) befindlichen Ausgleichseinrichtung (8) im Eingriff steht.

9. Endoskop gemäß einem der Ansprüche 3 bis 4, **dadurch gekennzeichnet, dass** der Endoskopschaft (3) eine in dessen Längsrichtung verlaufende Nut am Außenumfang hat, die weiter in einem in dem Endoskopschaft in dessen Längsrichtung ausgebildeten Kanal (11) verläuft, so dass eine durchgängige, geschlossene Nut (10) ausgebildet wird, in die der Stetigförderer (7) eingesetzt ist.

10. Endoskop gemäß einem der Ansprüche 1 bis 9, **dadurch gekennzeichnet, dass** der Endoskopschaft (3) zumindest eine optische Einrichtung hat, und wahlweise weiter eine Beleuchtungseinrichtung und/oder eine Behandlungseinrichtung und/oder eine Sprüheinrichtung hat.

## Claims

1. An endoscope comprising an everting tube (2) including a radially outer portion (5) and a radially inner portion (4), and an endoscope shaft (3) arranged at least partly in the everting tube, wherein longitudinal guiding means (6, 7) which engage with each other are formed at each of the endoscope shaft (3) and the everting tube(2), wherein
the longitudinal guiding means formed at the endoscope shaft is a continuous conveyor,
**characterized in that**
the continuous conveyor is in the form of a belt or strap and is guided in the longitudinal direction along the outside of the endoscope shaft.

2. The endoscope according to claim 1, **characterized in that** the longitudinal guiding means (6) formed at the everting tube (2) is an engaging means.

3. The endoscope according to claim 2, **characterized in that** a drive means (9) propels the radially inner portion (4) in the longitudinal direction thereof while the radially outer portion (5) at the same time is in a rest position, and a balancing means (8) arranged at the endoscope shaft (3) drives the continuous conveyor (7) engaged with the engaging means (3), whereby a movement of the endoscope shaft (3) relative to the everting tube (2) is controllable.

4. The endoscope according to claim 2, **characterized in that** a drive means (9) moves the endoscope shaft (3) in the longitudinal direction thereof, and a balancing means (8) arranged at the endoscope shaft (3) drives the continuous conveyor (7) engaged with the engaging means (6), whereby a movement of the radially inner portion (4) relative to the endoscope shaft (3) is controllable while the radially outer portion (5) at the same time is in a rest position.

5. The endoscope according to claim 2, **characterized in that** the continuous conveyor surrounds the everting tube and is preferably guided by rollers arranged at the two ends of the endoscope shaft which are wrapped, at least partly, preferably once or several times by the continuous conveyor.

6. The endoscope according to claim 5, **characterized in that** the continuous conveyor is further guided by a groove extending at an outer circumference of the endoscope shaft in a longitudinal direction thereof.

7. The endoscope according to claim 5 or 6, **characterized in that** a drive means either drives the continuous conveyor or moves the endoscope shaft forward in the longitudinal direction thereof.

8. The endoscope according to any one of claims 3 to 4, **characterized in that** the continuous conveyor (7) is guided by deflection rollers (9) disposed at the endoscope shaft (3), wherein a radially outer portion of the continuous conveyor (7) is engaged with the engaging means (6) disposed at the radially inner portion of the everting tube, and a radially inner portion of the continuous conveyor (7) is engaged with the balancing means (8) disposed at the endoscope shaft (3).

9. The endoscope according to any one of claims 3 to 4, **characterized in that** the endoscope shaft (3) includes a groove extending in the longitudinal direction thereof at the outer circumference which further extends in a passage (11) formed in the endoscope shaft in the longitudinal direction thereof so that a continuous closed groove (10) is formed into which the continuous conveyor (7) is inserted.

10. The endoscope according to any one of claims 1 to 9, **characterized in that** the endoscope shaft (3) has at least an optical means and optionally furthermore a lighting means and/or a means for treatment and/or a spraying means.

## Revendications

1. Endoscope, avec un tuyau flexible retourné (2), comportant une partie (5) radialement extérieure et une partie (4) radialement intérieure, et une tige d'endoscope (3) située au moins en partie dans le tuyau flexible retourné, des dispositifs de guidage longitudinal (6, 7) étant réalisés respectivement sur la tige d'endoscope (3) et le tuyau flexible retourné (2) et étant enclenchés l'un avec l'autre, le dispositif de guidage longitudinal, réalisé sur la tige d'endoscope, étant un transporteur continu,
**caractérisé en ce que** le transporteur continu est réalisé sous la forme d'une courroie ou bande et est guidé dans le sens longitudinal le long de la face extérieure de la tige d'endoscope.

2. Endoscope selon la revendication 1, **caractérisé en ce que** le dispositif de guidage longitudinal (6) réalisé sur le tuyau flexible retourné (2) est un dispositif d'enclenchement.

3. Endoscope selon la revendication 2, **caractérisé en ce qu'**un dispositif d'entraînement (9) fait avancer la partie (4) radialement intérieure dans le sens longitudinal de celle-ci alors que, en même temps, la partie (5) radialement extérieure est en position de repos, et un dispositif de compensation (8), situé sur la tige d'endoscope (3), actionne le transporteur continu (7) qui est en prise dans le dispositif d'enclenchement (3), moyennant quoi la tige d'endoscope (3) peut être commandée pour effectuer un mouvement relatif par rapport au tuyau flexible retourné (2).

4. Endoscope selon la revendication 2, **caractérisé en ce qu'**un dispositif d'entraînement (9) déplace la tige d'endoscope (3) dans le sens longitudinal de celle-ci et un dispositif de compensation (8), situé sur la tige d'endoscope (3), actionne le transporteur continu (7) qui est en prise dans le dispositif d'enclenchement (6), moyennant quoi la partie (4) radialement intérieure peut être commandée pour effectuer un mouvement relatif par rapport à la tige d'endoscope (3) alors que, en même temps, la partie (5) radialement extérieure est dans la position de repos.

5. Endoscope selon la revendication 2, **caractérisé en ce que** le transporteur continu entoure le tuyau flexible retourné et est guidé de préférence par des poulies, qui sont montées aux deux extrémités de la tige d'endoscope et autour desquelles le transporteur continu s'enroule au moins partiellement, de préférence une fois ou plusieurs fois.

6. Endoscope selon la revendication 5, **caractérisé en ce que** le transporteur continu est guidé, en outre, par une rainure réalisée sur le pourtour extérieur de la tige d'endoscope dans le sens longitudinal de celle-ci.

7. Endoscope selon la revendication 5 ou 6, **caractérisé en ce qu'**un dispositif d'entraînement actionne le transporteur continu ou fait avancer la tige d'endoscope dans le sens longitudinal de celle-ci.

8. Endoscope selon l'une quelconque des revendications 3 à 4, **caractérisé en ce que** le transporteur continu (7) est guidé par des poulies de renvoi (9), montées sur la tige d'endoscope (3), sachant qu'une partie radialement extérieure du transporteur continu (7) est en prise avec le dispositif d'enclenchement (6) situé sur la partie radialement intérieure du tuyau flexible retourné, et une partie radialement intérieure du transporteur continu (7) est en prise avec le dispositif de compensation (8) situé sur la tige d'endoscope (3).

9. Endoscope selon l'une quelconque des revendications 3 à 4, **caractérisé en ce que** la tige d'endoscope (3) comporte sur le pourtour extérieur une rainure qui s'étend dans le sens longitudinal de celle-ci, qui se prolonge, en outre, par un conduit (11) réalisé dans la tige d'endoscope dans le sens longitudinal de celle-ci, de telle sorte qu'il se forme une rainure (10) continue fermée, dans laquelle est inséré le transporteur continu (7).

10. Endoscope selon l'une quelconque des revendications 1 à 9, **caractérisé en ce que** la tige d'endoscope (3) comporte au moins un dispositif optique et, en outre, comporte au choix un dispositif d'éclairage et/ou un dispositif de traitement et/ou un dispositif de pulvérisation.
